# EUROPEAN PATENT SPECIFICATION

(11) **EP 0 687 169 B1**
(45) Date of publication and mention of the grant of the patent: **24.11.1999**
(21) Application number: 94909369.4
(22) Date of filing: 23.02.1994
(51) Int. Cl.: A61F 13/02, A61F 13/15

(54) **SURFACE MATERIAL FOR ABSORBENT ARTICLES**
OBERFLÄCHENMATERIAL FÜR ABSORBIERENDE ARTIKEL
MATERIAU DE SURFACE UTILISE DANS LES ARTICLES ABSORBANTS

(30) Priority: 01.03.1993 SE 9300683
(43) Date of publication of application: 20.12.1995
(73) Proprietor: Mölnlycke Health Care AB, 405 03 Göteborg (SE)
(72) Inventor: BILLGREN, Tomas, S-430 41 Kullavik (SE)
(74) Representative: Hyltner, Jan-Olof
(86) International application number: SE9400150
(87) International publication number: WO9420054

(56) References cited:
- EP-A- 0 057 484
- WO-A-91/11161
- US-A- 3 602 220
- US-A- 4 704 112

## Description

The present invention relates to surface material for absorbent articles, such as diapers, sanitary napkins, incontinence guards and wound dressings, said surface material being intended to lie proximal to the user's skin when the article is worn.

This type of surface material is intended to enhance the comfort with which the absorbent articles can be worn and so that the surface of the article that lies against the wearer's skin will be felt to be dry and comfortable. Functionally, this implies that the material shall not irritate the skin, that the surface layer shall coact with an absorbent body that lies outwardly in relation to the skin of the wearer in a manner such that fluid will be quickly transported away from the surface material and into the absorbent body, and that fluid absorbed by the body will be unable to pass back to that side of the surface material which is proximate to the skin. In addition, the surface material will also have a textile-like structure, since it has been found that the users of such articles prefer to have a textile-like material closest to the body.

In recent times, perforated plastic film has been used more and more as the surface material in absorbent sanitary products and absorbent hygiene products. One such known plastic film or sheet is comprised of a flat sheet in which there is formed a regular pattern of conical pits, each of which has a hole formed in the bottom surface thereof. This material thus has a flat side and a "spiky" side. When an absorbent article which includes such material is worn, the spiky side of the sheet faces towards the absorbent body of the article while the flat side lies against the wearer's skin. Such material can be manufactured cheaply and also provides a dry surface. The pits function as capillaries so that fluid discharged by the wearer will be sucked into the absorbent body, and the conical shape of the pits is considered to provide some sort of check-valve function which prevents fluid from passing back through the surface material when the article is subjected to pressure. One drawback with this surface material, however, is that the flat side of the material lies against the wearer's skin, meaning that moisture, such as perspiration, will be enclosed between the wearer's skin and the surface material, at least on the planar parts of the flat side, i.e. on those parts of the surface material which lie outside the pits.

The document US.A.3 602 220 discloses an outer covering formed of a sheet of flexible plastic material having therein openings in the form of hollow open-ended tunnellike portions upstanding from one face of the sheet and the document US.A.4 704 112 an apparatus comprising semi-spherical projections in order to form apertures in a web.

An object of the present invention is to provide an absorbent article surface material which, when the article is worn, will provide good ventilation between the wearer's skin and the surface material and which also has good properties in other respects.

This object is achieved in accordance with the invention in that surface material of the kind defined in the introduction is characterized in that said material includes a plurality of projections on that side thereof which is intended to lie proximal to the wearer's skin in use, and in that through-penetrating perforations are formed in those parts of the surface material which lie externally of the projections. Because only the tops of the projections lie against the wearer's skin, the major part of the surface material will be distanced from the skin of the wearer and ambient air is able to flow freely between the wearer's skin and the surface material. In addition, the total contact surface area of the surface material with the skin is small, which further enhances the comfort of a person which wears an absorbent article that includes such surface material.

According to one advantageous embodiment, the projections on the surface material have rounded tops, and the projections and the perforations are disposed in a regular pattern across essentially the total surface area of the surface material. The projections are also hollow, have a greater material thickness at their tops than at the remainder of the projections and have an upwardly narrowing shape. As a result of this configuration, the projections will lie in essentially punctiform contact with the wearer's skin, even when an absorbent article provided with inventive surface material is subjected to pressure, and the deformation forces will be distributed over a relatively large part of the walls of the projections.

The invention also relates to apparatus for manufacturing surface material for absorbent articles, such as diapers, sanitary napkins, incontinence guards and wound dressings, said surface material being intended to lie proximal to the wearer's skin when the article is worn, characterized by a mould which includes a regular pattern of conical or pyramidal bodies which project up from a smooth surface and which have rounded top surfaces, and in which holes are located in the smooth parts of the mould externally of the bodies, and which further includes means for placing on top of the mould a flat plastic sheet which has been heated to a mouldable state and means for generating a subpressure in the proximity of the holes in the mould.

The invention will now be described in more detail with reference to the accompanying drawings, in which
Fig. 1 illustrates part of a surface sheet constructed in accordance with one preferred embodiment of the invention;
Fig. 2 is a sectional view taken on the line II-II in Fig. 1;
Fig. 3 illustrates schematically apparatus for producing the surface material illustrated in Fig. 1;
Fig. 4 is a schematic perspective view of one embodiment of a mould that can be used for the manufacture of inventive surface material; and
Fig. 5 is a schematic sectional view of two mutually adjacent projections on the mould illustrated in Fig. 4.

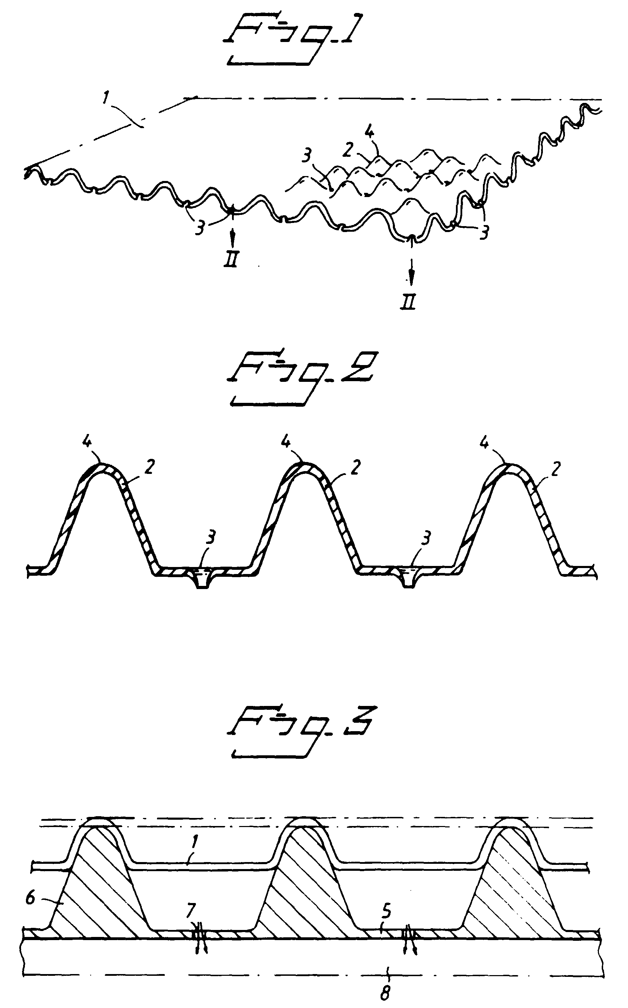

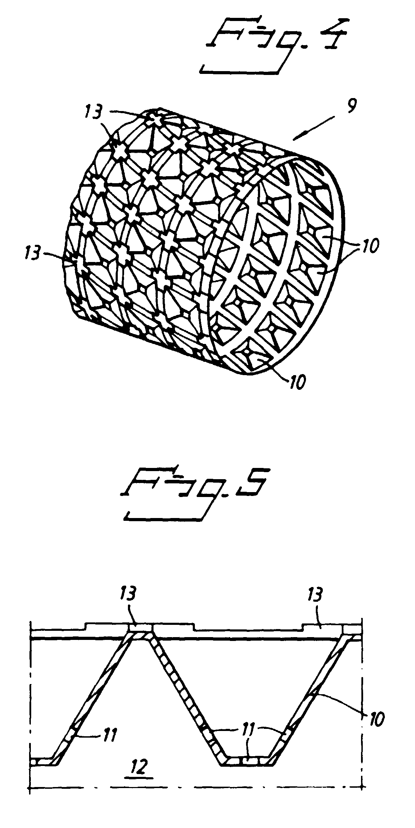

The surface material illustrated in Figures 1 and 2 is comprised of a plastic sheet 1, for instance a polyethylene sheet, which includes a plurality of conical projections 2 on one side thereof, as illustrated schematically in Figure 1. Perforations 3 are formed in the surface material in the flat regions between adjacent projections 2.

As mentioned earlier, the surface material is intended for use in absorbent one-time use articles, such as diapers, sanitary napkins, incontinence guards and wound dressings, with the side of the material on which the projections are provided being intended to face towards the wearer's skin and with the flat side of the material facing towards the absorbent body in the article concerned. When using an article which includes inventive surface material, only the tops 4 of the projections will lie against the wearer's skin. The surface material will thus abut the skin of the wearer solely in a punctiform fashion, and the skin contacting area of the surface material will therefore be very small, therewith preventing moisture from being enclosed between the skin and the surface material. Ambient air is also able to flow freely to the space between the surface material and skin, therewith purging this space of any moisture, such as perspiration, which may be present. Furthermore, when fluid is discharged in large quantities, such as urine, the absorbent body of the article coacting with the surface material is able to take-up the fluid in a conventional manner, because the fluid will flow into the areas between the projections on the surface material and through the perforations 3 in said areas and be absorbed by the absorbent body. The ventilation effect achieved with the inventive surface material will cause an absorbent article provided with such surface material to be felt very comfortable when worn.

When the inventive surface material is used in wound dressings, the ventilation effect will facilitate wound healing while the small total contact surface of the material will greatly reduce the tendency of the material to adhere to the wound.

The fluid permeability of the plastic sheet 1 is determined by the number and the size of the perforations 3, and the chosen ratio between open area per m² plastic sheet corresponds to the values of known perforated sheets intended for corresponding use.

In order for the surface material to function in the intended manner, the projections must be rigid enough to withstand the pressures to which the absorbent article comprising said surface material is normally subjected in use, without collapsing so as to block the air passageways between the projections. On the other hand, the projections must not be so rigid as to press hard against the wearer's skin when subjected to pressure and therewith feel uncomfortable.

In view of this, the projections 2 are hollow and the tops 4 of the projections 2 are preferably rounded, and even more preferable spherical in shape. In this way, when an absorbent article that is provided with surface material according to Figures 1 and 2 is subjected to pressure the forces acting on the wearer's skin will be distributed over a wider surface area than when the tops are pointed. The top 4 of a projection may also advantageously have a greater wall thickness than the remainder of the projection, which means that any deformation of the projection will begin away from the top and that deformation load will be distributed over a relatively large part of the projection wall via the top.

The height of the projection will preferably be chosen to be smaller than the diameter of a free droplet contained by the surface tension of the fluid to be absorbed by the absorbent body with which the surface material coacts, so as to ensure that droplets of discharged fluid will not remain in the projection interstices.

Figure 3 is a schematic cross-sectional view of part of an apparatus for producing the aforedescribed surface material. The apparatus includes a flat mould 5 which has a pattern of outwardly projecting conical bodies 6 whose outer contours are complementary to the shape of the projections 2 to be formed in an initially flat plastic sheet. Holes 7 are provided in the planar parts between the bodies 6 and are connected to a vacuum box 8.

When manufacturing inventive surface material, a plastic sheet 1 which has been heated to a mouldable state is placed on top of the mould 5 so as to rest on the tops of the upstanding bodies 6 of the mould, as indicated in chain lines in Figure 3. The underside of the plastic sheet is then subjected to subpressure with the aid of the vacuum box 8. The plastic sheet is sucked down towards the bottom of the mould 5 by the subpressure generated by the box 8 while being plastically deformed, until the sheet rests on the mould bottom. Plastic material is then drawn through the holes 7 in the mould and ruptures to form the aforesaid perforations 3. By appropriate dimensioning of manufacturing parameters, such as plastic sheet temperature and the value of the subpressure, surface material manufactured in this way can be given a greater wall thickness at the tops of respective projections than in the remainder of the projection walls, because the plastic sheet will begin to solidify at the tops of the mould body 6 immediately it is placed thereon.

The sheet manufacturing apparatus is preferably constructed to produce continuous webs of surface material, wherein the plastic sheet is applied to a mould according to Figure 3 formed on the periphery of a suction drum directly from an extrusion nozzle. The suction drum may be provided with two vacuum boxes arranged sequentially in the movement direction of the plastic sheet, of which boxes the downstream vacuum box will have a greater capacity than the upstream box so that the desired subpressure can be maintained even when sheet material has ruptured after having passes into the holes 7 in the mould.

Figures 4 and 5 illustrate schematically another mould 9 which can be used in the inventive apparatus and in which the holes are produced in the surface material either by punching or with the aid of an ultrasonic horn.

The mould 9 is also formed in the periphery of a suction drum, as illustrated in Figure 4 which illustrates schematically a section of the mould 9. Distinct from the mould 5 illustrated in Figure 3, the mould 9 includes bodies 10 which project in a regular pattern radially inwards from the periphery of a cylindrical surface instead of radially outwards as in the case of the mould 5. The bodies 10 have a truncated pyramidal configuration and the walls of said bodies include a plurality of holes 11 which open into the suction box 12 of the suction drum, although this has not been shown in Figure 4 for the sake of clarity. The base lines of the pyramids extend in an intersecting, diagonal pattern around the outer cylindrical surface of the mould 9 and the mould 9 has cruciform raised regions 13 between the pyramids at the points of intersection between the pyramid base lines. These raised regions 13 can be easily produced by machining down the outside of the mould 9 externally of the cruciform regions 13.

When using the mould 9 to produce inventive surface material, a sheet of plastic material in plastic state is placed on the mould and a subpressure is applied to the sheet so that the sheet will be drawn into the pyramidal bodies and therewith assume the shape of said bodies. No perforations are made in this stage of manufacture. The mouldable sheet is then caused to harden, optionally by supplying cooling air to the sheet. When the mouldable sheet has hardened, the sheet, while still on the mould 9, is caused to pass a counter-roller or an ultrasonic horn, so as to remove the material on the raised cruciform regions 13 of the mould and therewith form cruciform apertures in the hardened plastic sheet. The use of the mould 9 and a counter-roller or an ultrasonic horn when manufacturing the inventive surface material has the advantage that the amount of energy consumed and the amount of air that must be sucked away is less than when vacuum-forming the holes.

It is also conceivable to manufacture the surface material in a manner other than by vacuum-forming, for instance by passing the material between two moulds having mutually complementary projections and pits. It is also possible to begin with a flat plastic sheet and then heat the sheet to a mouldable state, instead of beginning with a newly extruded plastic melt.

It will be understood that the described and illustrated embodiment of the invention can be modified within the scope of the invention, particularly with respect to the shape of the projections and perforations and with respect to the patterns in which they are arranged. For instance, it is not necessary for that side of the surface material which lies proximal to the absorbent body to be flat, and it is also possible to use materials other than plastic sheet for the surface material. Because of the small contact surface between skin and surface material, not even hydrophilic material will be felt to be wet by a wearer. Plastic sheet is preferred, however, because of its low cost and good shapability. However, the pattern of projections, or possibly a combination of different patterns of projections, will preferably extend over the whole area of the surface material, so as to ensure good ventilation, whereas the pattern or patterns of perforations may be disposed solely in those parts of the surface material in which fluid can be expected to be discharged by the wearer. The invention is therefore only restricted by the content of the following Claims.

## Claims

1. Surface material (1) for absorbent articles, such as diapers, sanitary napkins, incontinence guards and wound dressings, said surface material being intended to lie nearest the wearer's skin in use, **characterized** in that the surface material (1) includes a plurality of projections (2) on that side thereof which lies proximal to the wearer's skin in use, and a plurality of through-penetrating perforations (3) in parts that lie outside the projections and in that the projections are rigid enough to withstand the pressures to which the absorbent article comprising said surface material is normally subjected in use, without collapsing, so that the surface material during normal use of the article abut the skin of a wearer essentially in a punctiform fashion.

2. Surface material according to Claim 1, **characterized** in that the projections (2) have rounded tops (4).

3. Surface material according to Claim 1 or 2, **characterized** in that the projections (2) and the perforations (3) are disposed in a regular pattern across essentially the whole area of the surface material.

4. Surface material according to any one of the preceding Claims, **characterized** in that the projections (2) are hollow and have a greater material thickness at the tops (4) thereof than in their remaining portions.

5. Surface material according to any one of the preceding Claims, **characterized** in that the projections (2) have an upwardly narrowing shape.

6. Apparatus for manufacturing surface material (1) for absorbent articles, such as diapers, sanitary napkins, incontinence guards and wound dressings, said surface material being intended to lie proximal to the wearer's skin in use, **characterized** by a mould (5) which includes a regular pattern of conical or pyramidal bodies (6) which project out from a smooth surface and which have rounded top surfaces; holes (7) in the smooth parts of the mould externally of the bodies (6); means for placing a flat plastic film heated to a mouldable state on top of the mould (5); and means (8) for generating a subpressure in the vicinity of the holes (7) in the mould.

7. Apparatus according to Claim 5, **characterized** in that the mould (5) extends around the periphery of a rotary drum with the holes (7) connecting with a vacuum box (8) located inwardly of the periphery of the drum; and in that the apparatus includes an extrusion nozzle which functions to lay plastic sheet in a mouldable state on top of the mould (5).

8. Apparatus for producing surface material (1) for absorbent articles such as diapers, sanitary napkins, incontinence guards and wound dressings, said surface material lying proximal to the wearer's skin in use, **characterized** by a cylindrical mould (9) which includes a regular pattern of conical or pyramidal bodies (10) which project radially inwards from the cylindrical surface of the mould and which have rounded top surfaces which include holes (11), and cruciform promontories (13) which project radially upwards from said cylindrical surface and which have a small radial extension in relation to said bodies and are located in parts of the cylindrical surface that lie outside the bodies (10) and are mutually spaced in peripheral and axial rows, a counter-roller which is intended to lie in a nip against the mould promontories (13); means for placing a flat plastic film heated to a mouldable state on top of the mould (9); and means (12) for generating a subpressure in the vicinity of the holes (11) in the mould.

## Patentansprüche

1. Oberflächenmaterial (1) für absorbierende Artikel, wie beispielsweise Windeln, Hygienebinden, Inkontinenzschutze und Wundverbände, wobei das Oberflächenmaterial dazu bestimmt ist, während des Gebrauchs nächst der Haut der Trägerperson zu liegen, **dadurch gekennzeichnet, daß** das Oberflächenmaterial (1) auf derjenigen Seite, die während des Gebrauchs zur Haut der Trägerperson proximal liegt, mehrere Vorsprünge (2) aufweist und in außerhalb der Vorsprünge liegenden Teilen mehrere durchgehende Durchbrüche (3) aufweist, und daß die Vorsprünge steif genug sind, um Drücken zu widerstehen, denen der das Oberflächenmaterial umfassende absorbierende Artikle normalerweise während des Gebrauchs unterworfen ist, ohne daß sie zusammenfallen, so daß das Oberflächenmaterial während des normalen Gebrauchs des Artikels an der Haut der Trägerperson in einer im wesentlichen punktförmigen Weise anliegt.

2. Oberflächenmaterial nach Anspruch 1, **dadurch gekennzeichnet, daß** die Vorsprünge (3) gerundete Spitzen (4) haben.

3. Oberflächenmaterial nach Anspruch 1 oder 2, **dadurch gekennzeichnet, daß** die Vorsprünge (2) und die Durchbrüche (3) über im wesentlichen die gesamte Fläche des Oberflächenmaterials in einem regelmäßigen Muster angeordnet sind.

4. Oberflächenmaterial nach einem der vorhergehenden Ansprüche, **dadurch gekennzeichnet, daß** die Vorsprünge (2) hohl sind und an den Spitzen (4) eine größere Materialdicke haben als in ihren restlichen Abschnitten.

5. Oberflächenmaterial nach einem der vorhergehenden Ansprüche, **dadurch gekennzeichnet, daß** die Vorsprünge (2) eine nach oben hin sich verschmälernde Form haben.

6. Vorrichtung zur Herstellung von Oberflächenmaterial (1) für absorbierende Artikel, wie beispielsweise Windeln, Hygienebinden, Inkontinenzschutze und Wundverbände, wobei das Oberflächenmaterial während des Gebrauchs dazu bestimmt ist, zur Haut der Trägerperson proximal zu liegen, **gekennzeichnet durch** eine Form (5), die ein regelmäßiges Muster konischer oder pyramidförmiger Körper (6) enthält, die von einer glatten Oberfläche vorstehen und gerundete Spitzenflächen aufweisen, Löcher (7) in den glatten Teilen der Form außerhalb der Körper (6), Mittel zum Plazieren einer flachen, auf einen formbaren Zustand erwärmten Kunststoffolie auf der Oberseite der Form (5) und Mittel (8) zum Erzeugen eines Unterdrucks in der Nähe der Löcher (7) in der Form.

7. Vorrichtung nach Anspruch 6, **dadurch gekennzeichnet, daß** sich die Form (5) um den Umfang einer Drehtrommel mit den Löchern (7) erstreckt, die mit einem innenseitig des Trommelumfangs liegenden Vakuumbehälter (8) verbunden ist, und daß die Vorrichtung eine Extrusionsdüse enthält, die dazu dient, eine Kunststofflage in einem formbaren Zustand auf der Oberseite der Form (5) aufzubringen.

8. Vorrichtung zur Herstellung von Oberflächenmaterial (1) für absorbierende Artikel, wie beispielsweise Windeln, Hygienebinden, Inkontinenzschutze und Wundverbände, wobei das Oberflächenmaterial beim Gebrauch zur Haut der Trägerperson proximal liegt, **gekennzeichnet durch** eine zylindrische Form (9), die ein regelmäßiges Muster aus konischen oder pyramidförmigen Körpern (10), welche von der Zylinderfläche der Form radial einwärts vorstehen und gerundete Spitzenflächen haben, die Löcher (11) aufweisen, und kreuzförmigen Erhebungen (13) enthält, die von der Zylinderfläche radial auswärts vorstehen und in bezug zu den Körpern eine kleinere radiale Erstreckung aufweisen und in Teilen der Zylinderfläche plaziert sind, die außerhalb der Körper (10) liegen, und in Umfangs- und Axialreihen beabstandet sind, eine Gegenwalze, die dazu bestimmt ist, in einem Spalt an die Formerhebungen (13) anzuliegen, Mittel zum Plazieren einer in einen formbaren Zustand erwärmten flachen Kunststoffolie auf der Oberseite der Form (9) und Mittel (12) zum Erzeugen eines Unterdrucks in der Nähe der Löcher (11) in der Form.

## Revendications

1. Matériau de surface (1) pour articles absorbants, tels que des couches-culottes, des serviettes hygiéniques, des protections pour incontinents et des pansements pour blessures, ledit matériau de surface étant destiné à être celui qui se trouve le plus près de la peau de l'utilisateur pendant l'utilisation, caractérisé en ce que le matériau de surface (1) comprend une multiplicité de saillies (2) sur son côté qui se trouve à proximité immédiate de la peau de l'utilisateur pendant l'utilisation, et une multiplicité de perforations traversantes (3) dans les parties qui se trouvent à l'extérieur des saillies, et en ce que les saillies sont suffisamment rigides pour supporter sans s'aplatir les pressions auxquelles est normalement soumis, pendant l'utilisation, l'article absorbant comprenant ledit matériau de surface, ce qui fait que le matériau de surface, pendant une utilisation normale de l'article, porte contre la peau d'un utilisateur essentiellement d'une façon multiponctuelle.

2. Matériau de surface selon la revendication 1, caractérisé en ce que les saillies (2) ont des sommets arrondis (4).

3. Matériau de surface selon la revendication 1 ou 2, caractérisé en ce que les saillies (2) et les perforations (3) sont disposées suivant un dessin régulier essentiellement sur la totalité de la superficie du matériau de surface.

4. Matériau de surface selon l'une quelconque des revendications précédentes, caractérisé en ce que les saillies (2) sont creuses et ont une épaisseur de matériau plus grande à leurs sommets (4) que dans leurs parties restantes.

5. Matériau de surface selon l'une quelconque des revendications précédentes, caractérisé en ce que les saillies (2) ont une forme qui devient de plus en plus étroite vers le haut.

6. Appareil de fabrication d'un matériau de surface (1) pour articles absorbants, tels que des couches-culottes, des serviettes hygiéniques, des protections pour incontinents et des pansements pour blessures, ledit matériau de surface étant destiné à être celui qui se trouve le plus près de la peau de l'utilisateur pendant l'utilisation, caractérisé par: un moule (5) qui comprend un dessin régulier de corps coniques ou pyramidaux (6) qui font saillie d'une surface lisse et qui comportent des surfaces supérieures arrondies; des trous (7) dans les parties lisses du moule extérieurement aux corps (6); des moyens pour placer sur le dessus du moule (5) un film plastique plat chauffé jusqu'à ce qu'il prenne un état moulable; et des moyens (8) pour générer une pression négative au voisinage des trous (7) dans le moule.

7. Appareil selon la revendication 6, caractérisé en ce que le moule (5) s'étend autour de la périphérie d'un tambour rotatif, le trous (7) étant raccordés à une boîte aspirante (8) placée intérieurement par rapport à la périphérie du tambour, et en ce que l'appareil comprend une buse d'extrusion qui fonctionne de manière à déposer une feuille de plastique dans un état moulable sur le dessus du moule (5).

8. Appareil de production de matériau de surface (1) destiné à des articles absorbants, tels que des couches-culottes, des serviettes hygiéniques, des protections pour incontinents et des pansements pour blessures, ledit matériau de surface se trouvant à proximité immédiate de la peau de l'utilisateur pendant l'utilisation, caractérisé par : un moule cylindrique (9) qui comprend un dessin régulier de corps coniques ou pyramidaux (10), qui font saillie radialement vers l'intérieur depuis la surface cylindrique du moule et qui comportent des surfaces supérieures arrondies comportant des trous (11), et des protubérances cruciformes (13) qui font saillie radialement vers le haut depuis ladite surface cylindrique et qui comportent une petite extension radiale par rapport auxdits corps et sont situées dans des parties de la surface cylindrique qui se trouvent à l'extérieur des corps (10) et sont mutuellement espacées en rangées périphériques et axiales; un rouleau d'appui qui est destiné à se trouver en contact avec les protubérances de moule (13); des moyens pour placer sur le dessus du moule (9) un film plastique plat chauffé jusqu'à ce qu'il prenne un état moulable; et des moyens (12) pour générer une pression négative au voisinage des trous (11) dans le moule.
